# EUROPEAN PATENT APPLICATION

(11) **EP 4 575 984 A1**
(43) Date of publication of application: **25.06.2025**
(21) Application number: 23854262.5
(22) Date of filing: 04.08.2023
(51) Int. Cl.: G06T 7/00

(54) **METHOD FOR AUTOMATICALLY IDENTIFYING AND POSITIONING PERFORATOR VESSEL, DEVICE, AND STORAGE MEDIUM**

(30) Priority: 15.08.2022 CN 202210972708
(71) Applicant: SHANGHAI NINTH PEOPLE'S HOSPITAL, SHANGHAI JIAOTONG UNIVERSITY SCHOOL OF MEDICINE, Huangpu District Shanghai 200011 (CN)
(72) Inventor: FENG, Shaoqing, Shanghai 200011 (CN); XI, Wenjing, Shanghai 200011 (CN); GU, Jianlei, Shanghai 200011 (CN); ZHANG, Yixin, Shanghai 200011 (CN)
(74) Representative: karo IP
(86) International application number: PCT/CN2023/111224
(87) International publication number: WO 2024/037358

(57) **Abstract**

The present invention relates to a method for automatically identifying and positioning a perforator vessel, a device, and a storage medium. The method comprises: according to a target perforator, determining a scanning range in a scanning direction, and extracting three-dimensional image data within the scanning range; performing dimension reduction processing on the three-dimensional image data to obtain layered segmented image data; taking the layered segmented image data as a minimum analysis unit, and screening for perforator vessel signal points by means of a signal-to-noise ratio dynamic threshold method; performing three-dimensional reconstruction on the perforator vessel by connecting the signal points in the three-dimensional image data, and screening for a candidate perforator vessel according to a signal-to-noise ratio total score after reconstruction; and determining a perforator vessel according to its relative position, its slope and depth to the epidermal layer and the deep muscle layer. The present invention can automatically and quickly position a perforator vessel.

## Description

### Technical field

The present invention relates to the technical field of medical image processing, and in particular to a method for automatically identifying and positioning perforator vessels.

### Background of the invention

Perforator flap transplantation surgery is at the forefront of the field of microsurgery and is an essential technology for body surface organ reconstruction (such as breast reconstruction), trauma or tumor defect repair to achieve the best repair effect and minimize donor site damage.

A skin flap refers to a composite tissue block containing skin and subcutaneous tissue, which contains its inherent feeding blood vessels. Skin flaps are often transferred from one part of the body to another to repair defects caused by the above reasons. This surgical procedure is called flap transplantation. Perforator vessels refer to blood vessels that penetrate the skin and its corresponding subcutaneous tissue. Their function is to provide blood supply to the skin they penetrate. The characteristic of the perforator flap is that the perforator vessels supplying the flap are completely dissected to the skin entry point, and the main blood vessels from which they originate are preserved. Other muscles and other tissues supplied by the main blood vessels are not affected. Traditional skin flap resection techniques do not dissect to the point of insertion, severing the vessel directly at the trunk, so the damage is greater.

The main technical difficulty of the perforator flap is the greater variability of perforator vessels, which not only exist in different human bodies, but also in different positions on both sides of the same body. Moreover, the caliber of the perforator vessel entry point is very thin (0.5-1mm), which is very easy to be damaged if the specific location is not known. Damage means the operation fails, so the operation risk is high and the surgeon is under great pressure during the operation.

The visualization imaging method is used to preoperatively locate the entry point of the perforator vessel, so that the surgeon can clearly understand the location of the entry point of the perforator vessel before surgery, which greatly reduces the risk and time of intraoperative dissection, thereby improving the success rate of surgery, among which medical imaging examinations of tomography (such as enhanced CT angiography, CTA, enhanced MR angiography, MRA) is one of the commonly used methods. The perforator vessel positioning technology of tomographic imaging scanning is relatively mature and has high positioning accuracy. The position information of the perforator vessels can be viewed from different dimensions. Volumetric reconstruction (VR) technology is then used to mark the entry points of the perforator vessels on the patient's body surface.

However, an important limitation of the above method is that the image reading and positioning work needs to be completed by the surgeons of the surgical team. Radiologists are unable to do so due to limited understanding of anatomy or other reasons, and there are few radiologists who can master this technology. The time take to read and mark tomographic images of a perforator vessel is usually about 45 minutes, which consumes a huge amount of energy for the surgeon and is a mechanical and repetitive task.

### Summary of the invention

The technical problem to be solved by the present invention is to provide a method for automatically identifying and positioning of perforator vessels, which can realize automatic and rapid positioning of perforator vessels.

The technical solution adopted by the present invention to solve the technical problem is to provide a method for automatically identifying and positioning method of perforator vessels, which includes the following steps:
according to a target perforator, determine the scanning range in the scanning direction, and extract three-dimensional image data within the scanning range;
perform dimension reduction processing on the three-dimensional image data to obtain layered segmented image data;
take the layered segmented image data as the minimum analysis unit, screen for the perforator vessel signal points by means of a signal-to-noise ratio dynamic threshold method;
perform three-dimensional reconstruction on the perforator vessel signal points connected in the three-dimensional image data, and screen for a candidate perforator vessel according to a signal-to-noise ratio total score after reconstruction;
determine the perforator vessels according to its relative position, its slope and depth to the epidermal layer and deep muscle layer, its slope, and depth.

The step of performing dimension reduction processing on the three-dimensional image data to obtain layered segmented image data specifically includes:
merge n consecutive images in the three-dimensional image data to obtain signal-enhanced two-dimensional image data;
mark the boundary lines of the skin surface and the boundary lines of subcutaneous fat and muscle in the two-dimensional image data, and use the subcutaneous fat area surrounded by the two boundary lines as the target analysis area;
divide the target analysis area into layers to obtain multiple layers image data of continuous subcutaneous depth.

When merging n consecutive images in the three-dimensional image data, the point with the smallest pixel value at the same position is taken as the pixel value of the merged image.

The boundary coordinates of the air and the skin surface are obtained through a machine learning algorithm, and the coordinates of the boundary line between the subcutaneous fat and the muscle are obtained through the pixel mean change rate.

The step of taking the layered segmented image data as the minimum analysis unit, screen for the perforator vessel signal points by means of a signal-to-noise ratio dynamic threshold method specifically includes:
convert the layered segmented image data of the same subcutaneous depth into a two-dimensional array, and select the minimum or maximum pixel value among several pixels at the same skin position and different depths in the two-dimensional array as the candidate blood vessel signal points, and take the minimum or maximum pixel value at its adjacent position as the background reference value to calculate the relative signal-to-noise ratio of the candidate blood vessel signal point;
select points with pixel values less than the pixel threshold as a set, and then select points from the set with relative signal-to-noise exceeding the signal-to-noise ratio threshold as perforator vessel signal points, and mark the corresponding abscissa of the perforating vessel signal points.

When calculating the relative signal-to-noise ratio of candidate blood vessel signal points, it is obtained by ((X₄+X₅/2)*(X₂+X₃+X₄+X₅))/(X₀+X₁), where Xₙ is the pixel mean value of points n pixels away from the candidate blood vessel signal point, and X₀ is the pixel value of the candidate blood vessel signal point.

The step of performing three-dimensional reconstruction on the perforator vessel signal points connected in the three-dimensional image data specifically includes:
map the perforator vessel signal point into the two-dimensional image data based on the corresponding abscissa, merge the points adjacent to the perforator vessel signal point in the two-dimensional image data, and calculate the continuity, spatial direction, and signal-to-noise ratio of the merged signal points of perforator vessels by summing;
the two-dimensional image data is fused to form a three-dimensional space, putting the merged points in the two-dimensional image data into the three-dimensional space, merging the points in the three-dimensional space adjacent to the merged points in the two-dimensional image data, and calculating the combined signal-to-noise ratio by summing.

After the step (5), it further includes: calculating the total score of each perforator vessel based on the signal-to-noise ratio and the blood vessel quality, and using the perforator vessel with the highest total score as the final perforator vessel.

The technical solution adopted by the present invention to solve the technical problem is to provide a computer device, including a memory and a processor. A computer program is stored in the memory. When the computer program is executed by the processor, causes the processor to perform the steps of the above-mentioned automatic identification and positioning method for perforator vessels.

The technical solution adopted by the present invention to solve its technical problems is: a computer-readable storage medium, on which an executable computer program is stored. When the executable computer program is executed by a processor, causes the processor to perform the steps of the above-mentioned automatic identification and positioning method of perforator vessels.

### Beneficial effects

Due to the adoption of the above technical solution, the present invention has the following advantages and positive effects compared with the prior art: the present invention searches for perforator signal points in the one-dimensional image by performing two dimensionality reduction processing on the three-dimensional image data. The processing speed is improved, allowing the perforator vessel positioning to be completed within 5 minutes. The method of the present invention uses the manual judgment results of physicians who are familiar with tomographic imaging perforator vessel positioning technology as the gold standard. The automated perforator vessel positioning results are basically consistent with the gold standard, and the identification rate of dominant perforator vessels exceeds 99%.

### Description of drawings

In order to explain the embodiments of the present invention or the technical solutions in the prior art more clearly, the drawings needed to be used in the description of the embodiments or the prior art will be briefly introduced below. Obviously, the drawings in the following description are only some embodiments of the present invention. For those skilled in the art, other drawings can be obtained based on these drawings without exerting creative efforts.
Figure 1 is a flow chart of the first embodiment of the present invention;
Figure 2 is a schematic diagram of the analysis range of two-dimensional image data in the first embodiment of the present invention;
Figure 3 is a schematic diagram of two-dimensional image data cut into long strips in the first embodiment of the present invention;
Figure 4 is a schematic diagram of converting two-dimensional image data into one-dimensional image data in the first embodiment of the present invention;
Figure 5 is a schematic diagram of the output result of single-layer two-dimensional image data in the first embodiment of the present invention;
Figure 6 is a schematic diagram of the result of merging consecutive points of different layers of two-dimensional image data in three-dimensional space in the first embodiment of the present invention;
Figure 7 is a schematic diagram of the distinction between subcutaneous veins and perforator vessels in the first embodiment of the present invention;
Figure 8 is a schematic diagram of preliminary comparison results between the calculation results of the first embodiment of the present invention and the gold standard;
Figure 9 is a schematic diagram of the human-computer interaction interface according to the second embodiment of the present invention.

### Preferred embodiments of the invention

The present invention will be further described below in conjunction with specific embodiments. It should be understood that these examples are only used to illustrate the invention and are not intended to limit the scope of the invention. In addition, it should be understood that after reading the teachings of the present invention, various changes or modifications may be made thereto by those skilled in the art, such equivalents falling within the scope of the invention as defined by the appended claims.

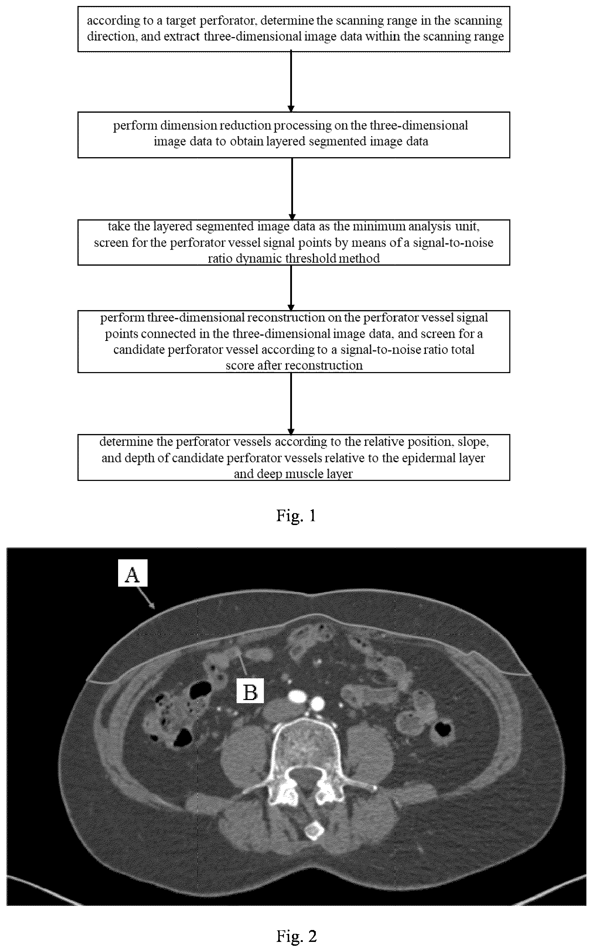

The embodiment of the present invention relates to a method for automatically identifying and positioning of perforator vessels, which can automatically analyze medical imaging data, automatically calculate and identify a target perforator vessel, ranking them according to their degree of dominance and returning their location information for use in preoperative perforator vessel positioning or other clinical and scientific applications requiring perforator vessel identification and positioning. As shown in Figure 1, the following steps are included: according to a target perforator, determine the scanning range in the scanning direction, and extract three-dimensional image data within the scanning range; perform dimension reduction processing on the three-dimensional image data to obtain layered segmented image data; take the layered segmented image data as the minimum analysis unit, screen for the perforator vessel signal points by means of a signal-to-noise ratio dynamic threshold method; perform three-dimensional reconstruction on the perforator vessel signal points connected in the three-dimensional image data, and screen for a candidate perforator vessel according to a signal-to-noise ratio total score after reconstruction; determine the perforator vessels according to the relative position, slope, and depth of candidate perforator vessels relative to the epidermal layer and deep muscle layer, and the coordinates of the perforator vessels in the three-dimensional coordinate system are returned.

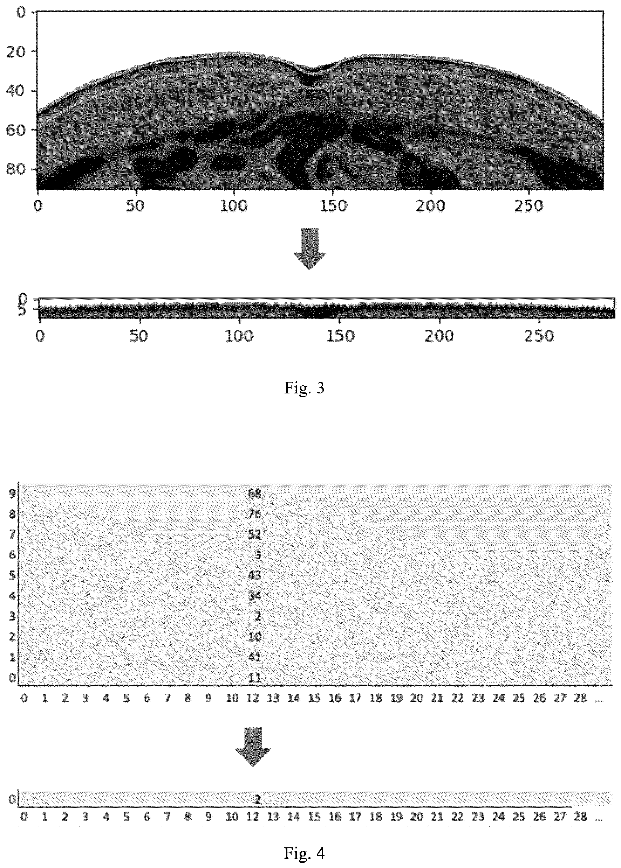

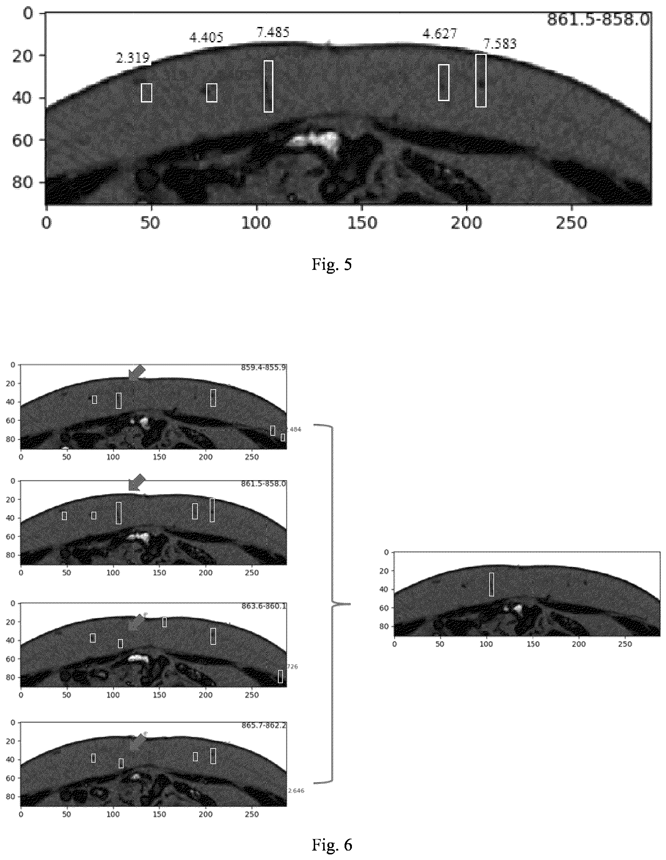

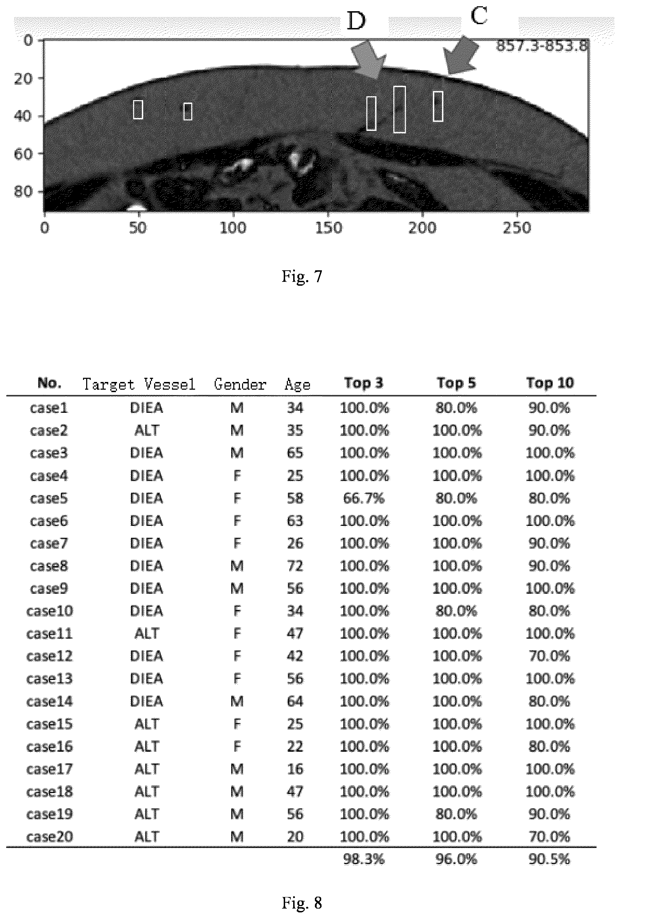

This method automatically reads and parses data in standardized tomographic medical imaging files (such as dicom); it can automatically identify the muscle exteriorization points of perforator vessels in the data and the travel paths within the subcutaneous soft tissue; and score and rank the perforator vessels based on information such as signal strength, direction of travel and the like, and the positioning information of the perforator vessels and their scores and ranking are output in the calculation results. Details, as follows:
1) Standardized tomographic imaging file data reading and analysis (the original image information is read into a multi-dimensional array, including the three-dimensional coordinates of the z, y, and x axes + the pixel value of the coordinates).
2) Limit the analysis range on the z-axis. According to the target perforator, the scanning range in the z-axis direction is limited, and the corresponding data is extracted. Take DIEA (inferior epigastric perforating artery) as an example, the original image covers the human body from the chest to the knees, DIEA vessels require analysis ranging from navel to above perineum, so based on the existing features of the image (navel will appear concave on cross-sectional images), the boundary of the body surface is identified first, then the location of the midpoint of the surface boundary is identified as the navel, and the dicom image from the belly circumference to the ulna is taken as the analysis object.
3) After appropriately merging the screenshots of adjacent horizontal planes on the z-axis, obtain two-dimensional image data, and analyze the two-dimensional image data. In order to enhance the signal of the perforator points, the subcutaneous perforator vessels run continuously, so the perforator signals on adjacent screenshots are generally adjacent in position, and after merging, they are basically continuous or fused. Number of horizontal screenshots in the z-axis direction after merging = number of horizontal screenshots in the previous intercept range/n (n = number of adjacent merged images). The merging logic in this step is: select the point with the smallest pixel value at the same position among n adjacent images as the pixel value of the merged image. The merged image is traversed, and a merged horizontal cross-sectional image (two-dimensional) is used as the unit for subsequent analysis, thereby converting the three-dimensional image into a two-dimensional image for analysis.
4) Limit the analysis range on the two-dimensional image, that is, mark the boundary lines between the air and the skin surface and the boundary lines between the subcutaneous fat and muscle in the two-dimensional image data, and use the area enclosed by two boundary lines as target analysis area.
   4.1 Upper bound of analytical range: in this step, the skin is defined, and the watershed algorithm marks the boundary between the air and skin surfaces.
   4.2 Lower bound of the analysis range: a) segment the two-dimensional image into strips, take the body surface shape as the side, read the image value with a height of 10 pixels, and put these values into a sub-image with a height of 10 and a width of the x-axis of the original image full length, reconstruct the sub-image from irregular shape to rectangular shape. b) Judgment of the lower bound of the analysis range: the inside of the abdominal cavity is similar to the outside of the body surface, with mainly dark colors, the pixel value of the subcutaneous tissue in the target analysis range is higher, setting a threshold for the segmented rectangular sub-image to determine the lower bound of the analysis range. In each of the above-mentioned segmented rectangular images with a height of 10 pixels, the mean value of the gray value is calculated: when the fraction of the point whose pixel value is higher closer to white reaches the threshold, the image has entered the range of the muscle, and is determined to be the lower bound of the analysis range has been reached. Figure 2 shows the analysis range of the two-dimensional image data in this embodiment. Figure 2A is the upper bound of the analysis range, B is the lower bound of the analysis range, and the target analysis area is between the two.
5) Segment the two-dimensional image within the analysis range into strips, and analyze each strip as a one-dimensional sequence. Call the method in 4.2 a) to cut the two-dimensional image into long strips with a height of 10 pixels, as shown in Figure 3. At this time, the two-dimensional image can be changed into a two-dimensional array, and then converted into a one-dimensional array, as shown in Figure 4. The data structure of the one-dimensional array is: [value corresponding to horizontal axis 1, value corresponding to horizontal axis 2,... ], the number of the elements in the array corresponds to the horizontal axis, and the corresponding value of the horizontal axis is: the smallest pixel value among the 10 pixels corresponding to the same horizontal axis (that is, the point with the strongest signal and the darkest color). Calculate the signal-to-noise ratio of the point with a "strong signal", in this embodiment, the signal-to-noise ratio is the comparison between the pixel value of the point itself and the pixel value of the surrounding points, the pixel value of the blood vessel is low, close to 0 (the color is dark in the image), and the surrounding points have high pixel values (closer to white in the image), therefore, the greater the difference between the signal point and the surrounding points, the more consistent it is with the characteristics of the blood vessels, and the higher the score. The signal-to-noise ratio calculation logic in this step is: ((X₄+X₅/2)*(X₂+X₃+X₄+X₅))/(X₀+X₁), where Xₙ is the pixel mean value of the point n pixels away from the perforator signal point, X₀ is the pixel value of the perforator signal point.
6) Find the "strong signal" point from the one-dimensional image data (the point closest to black in the image with the smallest pixel value), in this embodiment, the "strong signal" point is the perforator signal point, which can be found out in the following way: select points with pixel values less than 60 as a set, then select points with a relative signal-to-noise ratio greater than the median cutoff of the set as perforator signal points, and mark the corresponding abscissa of the perforator vessel signal point. If the abscissas corresponding to multiple perforator vessel signal points are too close together, they can be merged appropriately.
   Iterate the above steps 5)-6) until each two-dimensional image data is cut into layers and the calculation is completed.
7) Merge the adjacent points at the position of the "strong signal" point in the two-dimensional image, and add the signal-to-noise ratio of these points, that is, map the abscissa corresponding to the perforator vessel signal point to the two-dimensional image data, merge the points adjacent to the perforator vessel signal points in the two-dimensional image data, and sum the signal-to-noise ratios of these points, the continuity, spatial direction, and signal-to-noise ratio of the perforator vessel signal points after merging were obtained. Figure 5 is a schematic diagram of the output results of a single-layer two-dimensional image.
8) Put the points on the two-dimensional image in the previous step into three-dimension, merge adjacent points, and sum up the signal strengths. The signals of the same perforator will appear on different scanning levels, but their positional relationships are adjacent, therefore, in this step, the two-dimensional images are fused to form a three-dimensional space, and the previously identified points are put into the three-dimensional space for distance judgment, fuse adjacent points and sum the signal strengths (see Figure 6).
   It is worth mentioning that the thresholds in the above steps can be adjusted using real patient imaging data, and the best calculation results can be obtained after adjustment.
9) Distinguish subcutaneous veins and perforator vessels: high signal-to-noise ratio signals located next to each other may be subcutaneous veins as well as target perforator vessels. Both have higher signal intensity scores under the above steps, the main difference between the two is that the perforator vessels will continue to run deeper and eventually intersect with the muscle surface, while the subcutaneous veins always run parallel in the fat layer and do not intersect with the deep tissue (see Figure 7, where C is the perforator vessel and D is the venous signal). Therefore, in this step, the slope judgment logic and signal depth logic of the signal obtained in the previous step relative to the deep muscle layer are used to distinguish subcutaneous veins and perforator vessels, thereby eliminating venous signals.
10) After marking the perforator vessels, systematically score them to find the best perforator vessels. After excluding venous signals, there may be multiple perforator vessels, in order to ensure the success rate of the operation, this embodiment can score each perforator vessel and obtain the best perforator vessel as the final perforator vessel. In this step, when scoring, multiple perforator vessels are systematically scored based on the signal-to-noise ratio intensity of the perforator vessels and other determined logic that helps identify the quality of the vessels (such as outer diameter, bifurcation), the score can be calculated using a weighted summation method, after the score is calculated, the position of the candidate perforator signal with the highest score in the score is marked, and this position is the best identified perforator.
11) The coordinates of the muscle exit point of the best perforator vessel are converted to the body surface coordinate system, specifically: projecting the spatial position information of the muscle exit point of the perforator vessel onto the body surface, and a coordinate system is established with any marked point as the origin within the target range of the skin on the body surface, the algorithm calculates the coordinates of the projection point in the body surface coordinate system, which is convenient for users to mark its position information on the patient's body surface, providing support for intraoperative design.

The perforator vessels obtained using the above method are basically consistent with the gold standard, and the recognition rate exceeds 99%, the surgeon can review the automatically identified perforator images, confirm the results, and select the most matching candidate perforator vessels according to the needs of the surgical design. Among them, the gold standard is the manual judgment result of a physician who is familiar with the perforator vessel positioning technology of medical imaging in tomography.

Preliminary tests were conducted using 20 real case data (12 cases of inferior epigastric perforating vessels and 8 cases of descending lateral femoral artery perforator vessels), and the calculation results of this implementation method were compared with the average judgment results of three clinicians with >5 years of experience in manual identification of perforator vessels. The matching degree of the top 3 perforator vessels is 98.33%, the matching degree of the top 5 perforator vessels is 96%, and the matching degree of the top 10 perforator vessels is 90.50% (see Figure 8).

It is not difficult to find that the present invention can improve the processing speed by carrying out two dimensionality reduction processing on three-dimensional image data and finding the perforator signal points in one-dimensional images, so as to complete the perforator vessel positioning work within 5 minutes, compared with the original 45 minutes of judgment time, the perforator positioning work time has been greatly shortened and work efficiency has been improved.

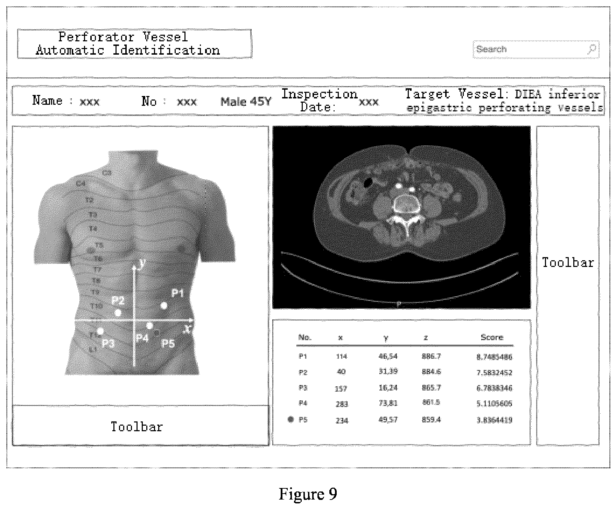

The second embodiment of the present invention relates to a computer device, including a memory and a processor, in which a computer program is stored, when the computer program is executed by the processor, the processor performs the steps of the perforator vessel automatic identification and localization method in the first embodiment. This embodiment can be installed on a variety of mobile computing platforms to realize mobile or remote operation of identification work (see Figure 9).

The third embodiment of the present invention relates to a computer-readable storage medium, an executable computer program is stored on the computer-readable storage medium, when the executable computer program is executed by a processor, it causes the processor to perform the steps of the automatic identification and positioning method for perforator vessels in the first embodiment.

## Claims

1. A method for automatically identifying and positioning perforator vessels, wherein, it comprises the following steps:
according to a target perforator, determine the scanning range in the scanning direction, and extract three-dimensional image data within the scanning range;
perform dimension reduction processing on the three-dimensional image data to obtain layered segmented image data;
take the layered segmented image data as the minimum analysis unit, screen for the perforator vessel signal points by means of a signal-to-noise ratio dynamic threshold method;
perform three-dimensional reconstruction on the perforator vessel by connecting the signal points in the three-dimensional image data, and screen for a candidate perforator vessel according to a signal-to-noise ratio total score after reconstruction;
determine the perforator vessel according to its relative position, its slope and depth to the epidermal layer and deep muscle layer.

2. The method of claim 1, wherein, the step of performing dimension reduction processing on the three-dimensional image data to obtain layered segmented image data specifically includes:
merge n consecutive images in the three-dimensional image data to obtain signal-enhanced two-dimensional image data;
mark the boundary lines of the skin surface and the boundary lines of subcutaneous fat and muscle in the two-dimensional image data, and use the subcutaneous fat area surrounded by the two boundary lines as the target analysis area;
divide the target analysis area into layers to obtain multiple layers image data of continuous subcutaneous depth.

3. The method of claim 2, wherein, when merging n consecutive images in the three-dimensional image data, the point with the smallest pixel value at the same position is taken as the pixel value of the merged image.

4. The method of claim 2, wherein, the boundary coordinates of the air and the skin surface are obtained through a machine learning algorithm, and the coordinates of the boundary line between the subcutaneous fat and the muscle are obtained through the pixel mean change rate.

5. The method of claim 1, wherein, the step of taking the layered segmented image data as the minimum analysis unit, screen for the perforator vessel signal points by means of a signal-to-noise ratio dynamic threshold method specifically includes:
convert the layered segmented image data of the same subcutaneous depth into a two-dimensional array, and select the minimum or maximum pixel value among several pixels at the same skin position and different depths in the two-dimensional array as the candidate blood vessel signal points, and take the minimum or maximum pixel value at its adjacent position as the background reference value to calculate the relative signal-to-noise ratio of the candidate blood vessel signal point;
select points with pixel values less than the pixel threshold as a set, and then select points from the set with relative signal-to-noise exceeding the signal-to-noise ratio threshold as perforator vessel signal points, and mark the corresponding abscissa of the perforator vessel signal points.

6. The method of claim 5, wherein, when calculating the relative signal-to-noise ratio of candidate blood vessel signal points, it is obtained by ((X₄+X₅/2)*(X₂+X₃+X₄+X₅))/(X₀+X₁), where Xₙ is the pixel mean value of points n pixels away from the candidate blood vessel signal point, and X₀ is the pixel value of the candidate blood vessel signal point.

7. The method of claim 1, wherein, the step of performing three-dimensional reconstruction on the perforator vessel signal points connected in the three-dimensional image data specifically includes:
map the perforator vessel signal point into the two-dimensional image data based on the corresponding abscissa, merge the points adjacent to the perforator vessel signal point in the two-dimensional image data, and calculate the continuity, spatial direction, and signal-to-noise ratio of the merged signal points of perforator vessels by summing;
the two-dimensional image data is fused to form a three-dimensional space, put the merged points in the two-dimensional image data into the three-dimensional space, merge the points in the three-dimensional space adjacent to the merged points in the two-dimensional image data, and calculate the combined signal-to-noise ratio by summing.

8. The method of claim 1, wherein, after step (5), it further includes: calculating the total score of each perforator vessel based on the signal-to-noise ratio and the blood vessel quality, and using the perforator vessel with the highest total score as the final perforator vessel.

9. A computer device, wherein, it includes a memory and a processor, a computer program is stored in the memory, when the computer program is executed by the processor, the processor performs the steps of the automatic identification and positioning method of the perforator vessels as described in claim 1.

10. A computer-readable storage medium, wherein, an executable computer program is stored on the computer-readable storage medium, and when the executable computer program is executed by a processor, the processor performs the steps of the perforator vessel automatic identification and positioning method described in claim 1.
